# EUROPÄISCHE PATENTSCHRIFT

(11) **EP 0 834 503 B1**
(45) Veröffentlichungstag und Bekanntmachung des Hinweises auf die Patenterteilung: **27.06.2001**
(21) Anmeldenummer: 97118857.8
(22) Anmeldetag: 20.04.1993
(51) Int. Cl.: C07D 209/48, A01N 37/32

(54) **Tetrahydrophthalimide, deren Herstellung und Verwendung**
Tetrahydrophthalimides, their preparation and use
Tétrahydrophtalimides, leur préparation et utilisation

(30) Priorität: 25.04.1992 DE 4213715; 11.11.1992 DE 4238001
(43) Veröffentlichungstag der Anmeldung: 08.04.1998
(62) Teilanmeldung aus: 93909375.3
(73) Patentinhaber: BASF AKTIENGESELLSCHAFT, 67056 Ludwigshafen (DE)
(72) Erfinder: Klintz, Ralf Dr., 4630 Bochum (DE); Heistracher, Elisabeth Dr., 68159 Mannheim (DE); Schaefer, Peter Dr., 67308 Ottersheim (DE); Hamprecht, Gerhard Dr., 6940 Weinheim (DE); Plath, Peter Dr., 6710 Frankenthal (DE); Kardorff, Uwe Dr., 6800 Mannheim 1 (DE); Gerber, Matthias Dr., 6703 Limburgerhof (DE); Westphalen, Karl-Otto Dr., 6720 Speyer (DE); Walter, Helmut Dr., 6719 Obrigheim (DE)

(56) Entgegenhaltungen:
- EP-A- 0 240 659
- EP-A- 0 320 677
- EP-A- 0 385 231
- DE-A- 4 237 984

## Beschreibung

Die vorliegende Erfindung betrifft neue substituierte 3,4,5,6-Tetrahydrophthalimide der allgemeinen Formel I 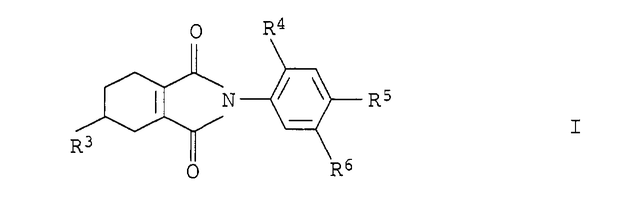 in der die Substituenten folgende Bedeutung haben:
- R³: Wasserstoff oder eine Methylgruppe;
- R⁴: Fluor;
- R⁵: Chlor;
- R⁶: -CH₂-CHR¹CO₂R²;
- R¹: Chlor, Brom;
- R²: Methyl, Ethyl;

Es ist bekannt, daß substituierte 3,4,5,6-Tetrahydrophthalimide als Herbizide (vgl. DE-A 36 03 789, EP-A 300 398) bzw. als Desikkations- und Abzissionsmittel (vgl. DE-A 39 05 916) verwendet werden. Ihre Wirkung ist jedoch unbefriedigend.

Demgemäß wurden die voranstehend definierten 3,4,5,6-Tetrahydrophthalimide der allgemeinen Formel I gefunden.

Ferner wurden herbizide Mittel und Mittel zur Defoliation bzw. Desikkation gefunden, die diese Substanzen I enthalten.

Die substituierten 3,4,5,6-Tetrahydrophthalimide der Formel I können in Form ihrer umweltverträglichen Salze vorliegen, wobei im allgemeinen die Salze von solchen Basen in Betracht kommen, die die herbizide Wirkung von I nicht beeinträchtigen.

Als basische Salze eignen sich besonders diejenigen der Alkalimetalle, vorzugsweise Natrium- und Kaliumsalze, die der Erdalkalimetalle, vorzugsweise Calcium- und Magnesiumsalze und die Übergangsmetalle, vorzugsweise Zink- und Eisensalze, sowie der Ammoniumsalze, die ein bis drei C₁-C₄-Alkyl, Hydroxy-C₁-C₄-Alkyl-substituenten und/oder einen Phenyl- oder Benzylsubstituenten tragen können, vorzugsweise Diisopropylammonium-, Tetramethylammonium-, Tetrabutylammonium-, Trimethylbenzylammonium- und Trimethyl-(2-hydroxyethyl)-ammoniumsalze, die Phosphonium-, Sulfonium- und Sulfoxoniumsalze.

Die Verbindungen der Formel I können ein Chiralitätszentrum enthalten und liegen dann als Enantiomerengemische vor. Die Erfindung umfaßt sowohl die reinen Enantiomeren als auch deren Gemische.

Im Hinblick auf die Verwendung der erfindungsgemäßen substituierten 3,4,5,6-Tetrahydrophthalimide der Formel I als herbizide und/ oder defoliant/desikkant wirksame Verbindungen sind in nachfolgender Tabelle 1 besonders bevorzugte Verbindungen aufgeführt:

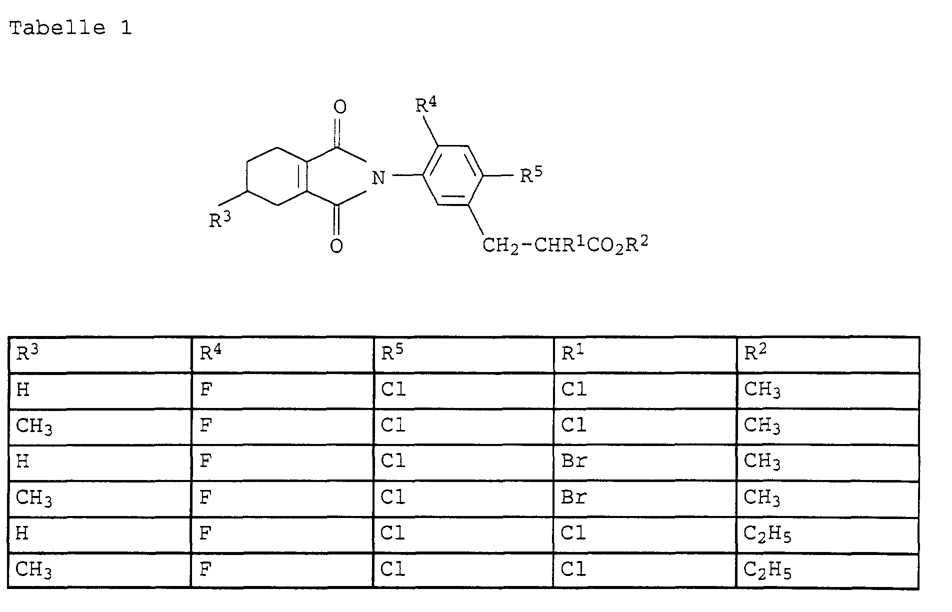

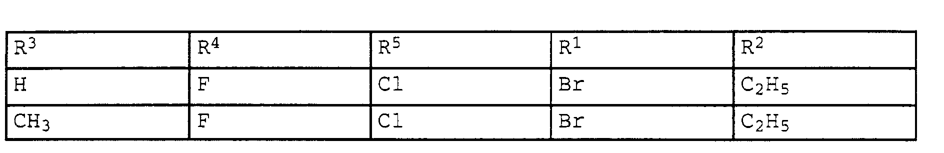

Die Verbindungen der Formel I sind auf vielfältige Weise analog zu bekannten Umsetzungsmethoden erhältlich. Exemplarisch seien im folgenden einige Verfahren erläutert:

### Verfahren A

Man erhält Verbindungen der Formel I in an sich bekannter Art und Weise (H.P. Doyle, B. Siegfried, P.C. Elliott, J.F. Dellariar, J. Org. Chem. 42, 2431 (1977)) durch Umsetzung einer Verbindung der Formel III und einer Verbindung der Formel IV im Rahmen einer Meerwein'schen Arylierungsreaktion oder einer üblichen Modifikationen hiervon.

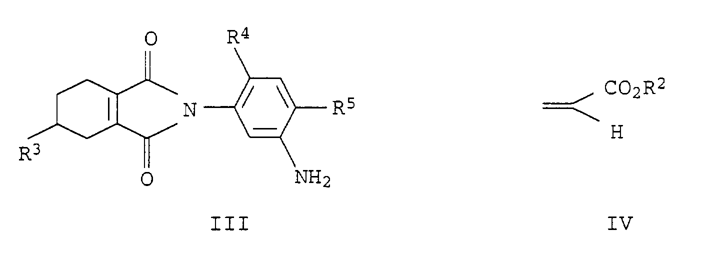

Bei diesem Reaktionstyp wird die "Aminoverbindung" in ein Diazoniumsalz übergeführt. Dieses reagiert mit einem Olefin in Gegenwart eines Kupfersalzes ab.

Zweckmäßigerweise wird das "Phenyldiazoniumsalz" in an sich bekannter Art und Weise in wäßriger Säurelösung, wie z.B. Salzsäure, Bromwasserstoffsäure, Schwefelsäure durch Umsetzung einer Aminoverbindung der Formel III mit einem Nitrit wie z.B. Natriumnitrit, Kaliumnitrit etc. erhalten. Anschließend wird die ungesättigte Komponente IV in einem geeigneten Lösungsmittel wie z.B. H₂O, Aceton, Diethylketon, Methylethylketon, Acetonitril, Dioxan, THF, Methanol, Ethanol etc., in Gegenwart eines Kupferhalogenides, wie z.B. CuCl, CuBr, CuCl₂, CuBr₂ zugegeben. Die Umsetzungen können bei Temperaturen von -30°C bis +50°C durchgeführt werden. Üblicherweise werden die Komponenten der Diazotierungsreaktion im stöchiometrischen Verhältnis eingesetzt, jedoch kann ein Überschuß der einen oder anderen Komponente von Vorteil sein.

In der Regel werden Verbindungen der Formel IV im großen Überschuß eingesetzt, es kann aber auch von Vorteil sein, sie im kleinen Überschuß, stöchiometrisch oder im Unterschuß einzusetzen.

Das Kupferhalogenid wird in der Regel im stöchiometrischen Verhältnis eingesetzt, jedoch kann ein Über- oder Unterschuß von Vorteil sein.

Alternativ kann das "Phenyldiazoniumsalz" in an sich bekannter Art und Weise in wasserfreien Systemen, wie z.B. chlorwasserstoffhaltiger Eisessig, Dioxan, abs. Alkohol, THF, Acetonitril, Aceton mit einem Salpetrigsäureester, wie z.B. tert.-Butylnitrit, Isopentylnitrit, etc. erhalten werden. Die Diazotierung kann in Gegenwart der Olefinkomponente IV und des Kupferhalogenides stattfinden oder vor Zugabe der beiden letztgenannten Komponenten.

### Verfahren B

Man erhält Verbindungen der Formel I in an sich bekannter Art und Weise durch Hydrierung von Verbindungen der Formel V

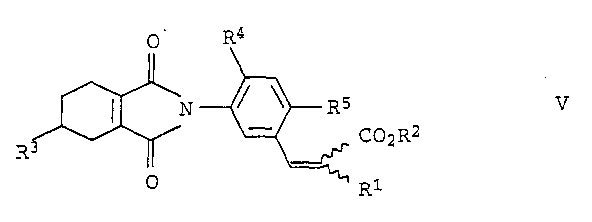

Als Reduktionsmittel kommen z.B. elementare Metalle, wie z.B. Eisen, Zinn, Zink etc., Wasserstoff in Gegenwart von geeigneten Katalysatoren, wie z.B. Pd/C, Pt/C, Raney-Ni etc., komplexe Metallhydride wie z.B. LiAlH₄, NaBH₄, etc., ggf. in Gegenwart von Katalysatoren, in Betracht.

Als Lösungsmittel verwendet man in Abstimmung mit dem Reduktionsmittel üblicherweise Säuren, wie z.B. Essigsäure, Propionsäure etc., Alkohole wie z.B. Methanol, Ethanol etc., Ether wie z.B. Diethylether, Methyl-tert.-Butylether, THF, Dioxan etc., Aromaten wie z.B. Benzol, Toluol etc. oder entsprechende Gemische.

Die Umsetzungen können bei Temperaturen von -100°C bis Rückflußtemperatur des jeweiligen Lösungsmittels bzw. -gemisches durchgeführt werden.

Üblicherweise werden die Edukte im stöchiometrischen Verhältnis eingesetzt, jedoch kann in Einzelfällen ein Überschuß der einen oder anderen Komponente von Vorteil sein.

### Verfahren C

Man erhält Verbindungen der Formel I nach literaturbekannten, bzw. in Analogie zu literaturbekannten Verfahren aus Verbindungen der Formel I⁺ durch Austausch von "R¹⁺" durch ein anderes Halogenid, wobei die Edukte nach den Verfahren A, B und D erhalten werden.

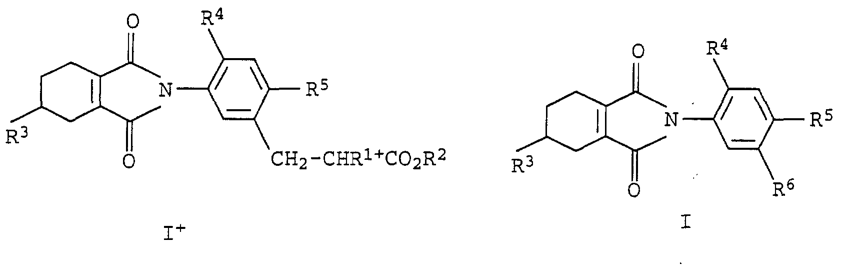

Als "R¹⁺" kommen Chlor oder Brom in Betracht als "R¹" Brom oder Chlor, wobei Nickelkatalysatoren eingesetzt werden, in entsprechend abgestimmten aprotischen Lösungsmittel- bzw. -gemischen wie Aceton, Diethylketon, Methylethylketon, Dimethylformamid, Diethylformamid, N-Methylpyrrolidon, Dimethylsulfoxid, Sulfolan, chlorierte Kohlewnasserstoffe wie Methylenchlorid, Chloroform etc.

Die Umsetzungen werden gewöhnlich in einem Temperaturbereich von -30°C bis Rückflußtemperatur des jeweiligen Lösungsmittels bzw. -gemisches durchgeführt.

In der Regel werden die Ausgangsstoffe in stöchiometrischem Verhältnis eingesetzt, jedoch kann ein Über- bzw. Unterschuß der einen oder anderen Komponente von Vorteil sein.

### Verfahren D

Man erhält Verbindungen der Formel I in an sich bekannter Weise durch Kondensation eines Anhydrids der Formel II und einem Anilin der Formel VI 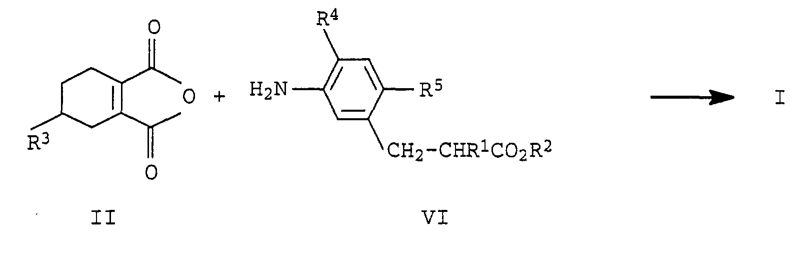 in einem inerten organischen Lösungsmittel. Hierfür eignen sich Alkancarbonsäuren wie z.B. Essigsäure, Propionsäure, Isobuttersäure, Alkancarbonsäureester wie z.B. Essigester, aprotischen Solventien, wie z.B. Dimethylformamid, Dimethylsulfoxid, N-Methylpyrrolidon etc., Aromaten wie z.B. Toluol, Xylol etc. Bei Verwendung eines aprotischen Lösungsmittels empfiehlt sich die kontinuierliche Entfernung des entstehenden Reaktionswassers bzw. eine Säurekatalyse, so z.B. durch p-Toluolsulfonsäure, (Trifluor)methylsulfonsäure etc..

Die Umsetzungen werden gewöhnlich in einem Temperaturbereich von 0°C bis Rückflußtemperatur des Lösungsmittels bzw. -gemisches durchgeführt.

In der Regel werden die Ausgangsstoffe im stöchiometrischen Verhältnis eingesetzt. Jedoch kann ein Über- bzw. Unterschuß der einen oder anderen Komponente von Vorteil sein.

### Verfahren E

Man erhält Verbindungen der Formel VI in an sich bekannter Art und Weise (Houben-Weyl, Bd. XI/1, S. 341 ff (4. Auflage)) durch Reduktion der entsprechenden Nitroverbindung VII.

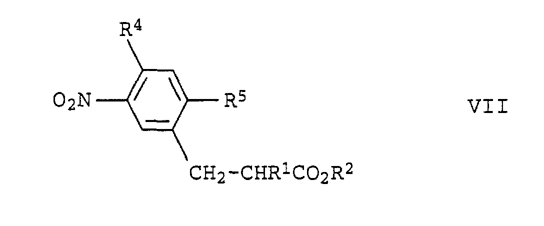

Als Reduktionsmittel kommen z.B. elementare Metalle, wie z.B. Eisen, Zinn, Zink etc., Wasserstoff in Gegenwart von geeigneten Katalysatoren, wie z.B. Pd/C, Pt/C, Raney-Ni etc., komplexe Metallhydride wie z.B. LiAlH₄, NaBH₄, etc., ggf. in Gegenwart von Katalysatoren in Betracht.

Als Lösungsmittel verwendet man in Abstimmung mit dem Reduktionsmittel üblicherweise Säuren, wie z.B. Essigsäure, Propionsäure etc., Alkohole wie z.B. Methanol, Ethanol etc., Ether wie z.B. Diethylether, Methyl-tert.-Butylether, THF, Dioxan etc., Aromaten wie z.B. Benzol, Toluol etc. oder entsprechende Gemische.

Die Umsetzungen können bei Temperaturen von -100°C bis Rückflußtemperatur des jeweiligen Lösungsmittels bzw. -gemisches durchgeführt werden.

Üblicherweise werden die Edukte im stöchiometrischen Verhältnis eingesetzt, jedoch kann in Einzelfällen ein Überschuß der einen oder anderen Komponente von Vorteil sein.

### Verfahren F

Man erhält Verbindungen der Formel VII mit in an sich bekannter Art und Weise (M.P. Doyle, B. Siegried, R.C. Elliot, J.F. Dellaria, J. Org. Chem., 42, 2431 (1977)) durch Umsetzung eines Anilins der Formel VIII und einer Verbindung der Formel IV im Rahmen einer Meerwein'schen Arylierungsreaktion oder Modifikationen hiervon.

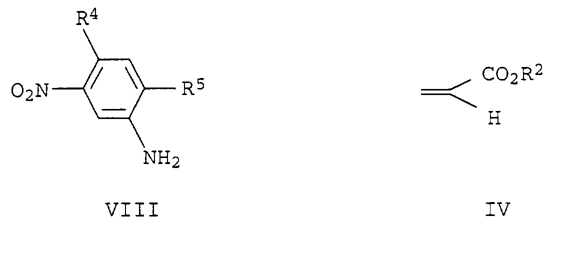

Bei diesem Reaktionstyp wird die "Aminoverbindung" in ein Diazoniumsalz übergeführt. Dieses reagiert mit einem Olefin in Gegenwart eines Kupfersalzes ab.

Zweckmäßigerweise wird das "Phenyldiazoniumsalz" in an sich bekannter Art und Weise in wäßriger Säurelösung, wie z.B. Salzsäure, Bromwasserstoffsäure, Schwefelsäure durch Umsetzung einer Aminoverbindung der Formel VIII mit einem Nitrit wie z.B. Natriumnitrit, Kaliumnitrit etc. erhalten. Anschließend wird die ungesättigte Komponente IV in einem geeigneten Lösungsmittel wie z.B. H₂O, Aceton, Diethylketon, Methylethylketon, Acetonitril, Dioxan, THF, Methanol, Ethanol etc., in Gegenwart eines Kupferhalogenides, wie z.B. CuCl, CuBr, CuCl₂, CuBr₂ zugegeben. Die Umsetzungen können bei Temperaturen von -30°C bis +50°C durchgeführt werden. Üblicherweise werden die Komponenten der Diazotierungsreaktion im stöchiometrischen Verhältnis eingesetzt, jedoch kann ein Überschuß der einen oder anderen Komponente von Vorteil sein.

In der Regel werden Verbindungen der Formel IV im großen Überschuß eingesetzt, es kann aber auch von Vorteil sein, sie im kleinen Überschuß, stöchiometrische oder im Unterschuß einzusetzen.

Das Kupferhalogenid wird in der Regel im stöchiometrischen Verhältnis eingesetzt, jedoch kann ein Über- oder Unterschuß von Vorteil sein.

Alternativ kann das "Phenyldiazoniumsalz" in an sich bekannter Art und Weise in wasserfreien Systemen, wie z.B. chlorwasserstoffhaltiger Eisessig, Dioxan, abs. Alkohol, THF, Acetonitril, Aceton mit einem Salpetrigsäureester, wie z.B. tert.-Butylnitrit, Isopentylnitrit, etc. erhalten werden. Die Diazotierung kann in Gegenwart der Olefinkomponente IV und des Kupferhalogenides stattfinden oder vor Zugabe der beiden letztgenannten Komponenten.

Die Tetrahydrophthalimide I können bei der Herstellung als Isomerengemische anfallen. Alle Isomerengemische lassen sich jedoch gewünschtenfalls nach den hierfür üblichen Methoden, z.B. durch Kristallisation oder Chromatographie, gegebenenfalls an einem optisch aktiven Absorbat, in die reinen Isomere trennen.

Mischungen aus den optisch aktiven Isomeren, die ein Isomeres im Überschuß enthalten, lassen sich beispielsweise auch unter Verwendung optisch aktiver Ausgangsprodukte herstellen.

Die erfindungsgemäßen substituierten Tetrahydrophthalimide I eignen sich, sowohl als Isomerengemische als auch in Form der reinen Isomeren, als Herbizide und als Defoliations-/Desikkationsmittel.

Die substituierten Tetrahydrophthalimide I eignen sich, sowohl als Isomerengemische als auch in Form der reinen Isomeren, als Herbizide, insbesondere zur Bekämpfung von dikotylen Unkräutern.

Insbesondere bei niedrigen Aufwandmengen sind sie verträglich und somit selektiv in Kulturen wie Weizen, Reis, Mais, Soja und Baumwolle.

Die substituierten Tetrahydrophthalimide I eignen sich außerdem als Desikkantien und Defoliantien, insbesondere zur Entlaubung von Baumwolle, und als Defoliantien zur Austrocknung der oberirdischen Pflanzenteile bei Kulturpflanzen, z.B. Kartoffel, Sonnenblume, Sojabohne und Raps. Damit wird ein vollständig mechanisiertes Beernten dieser wichtigen Kulturpflanzen ermöglicht.

Von wirtschaftlichem Interesse ist ferner die Ernteerleichterung,die durch das zeitlich konzentrierte Abfallen der oder Vermindern der Haftfestigkeit am Baum bei Zitrusfrüchten, Oliven oder bei anderen Arten und Sorten von Kern-, Stein- und Schalenobst ermöglicht wird. Außerdem führen sie zu einer gleichmäßigen Abreife der Erntefrüchte.

Derselbe Mechanismus, d.h. die Förderung der Ausbildung von Trenngewebe zwischen Frucht- bzw. Blatt- und Sproßteil der Pflanze ist auch für ein gut kontrollierbares Entblättern von Nutzpflanzen wie insbesondere Baumwolle wesentlich. Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden herbiziden oder Desikkations-/Defoliations-Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulosein Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Beispiele für solche Zubereitungen sind:
I. eine Mischung aus 20 Gew.-Teilen der Verbindung (N-[4-Chlor-3-(2-chlor-2-ethoxycarbonyl-ethenyl)-phenyl]-3,4,5,6-tetrahydrophthaldiamid)*, 80 Gew.-Teilen Xylol, 10 Gew.-Teilen des Anlagerungsproduktes von 8 bis 10 Mol Ethyle-noxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine wäßrige Dispersion, die 0,02 Gew.-% des Wirkstoffs enthält.
II. eine Dispersion von 20 Gew.-Teilen der Verbindung (N,N-Dimethyl-N'-[4-chlor-3-(2-chlor-2-ethoxycarbonyl-ethenyl)-phenyl]-3,4,5,6-tetrahydrophthaldiamid)*, in einer Mischung aus 40 Gew.-Teilen Cyclohexanon, 30 Gew.-Teilen Isobutanol, 20 Gew.-Teilen des Anlagerungsproduktes von 7 mol Ethylenoxid an 1 mol Isooctylphenol und 10 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.
III. eine Dispersion von 20 Gew.-Teilen der Verbindung (2-(N-Pyrrolidinocarbonyl)-1-N'-[4-chlor-3-(2-chlor-2-ethoxycarbonylethenyl)-phenyl]-aminocarbonylcyclohexen)*, in einer Mischung aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;
IV. eine in einer Hammermühle vermahlene Mischung aus 20 Gew.-Teilen der Verbindung (N-[4-Chlor-5-(2-chlor-2-methoxycarbonylethyl-1)-phenyl]-3,4,5,6-tetrahydrophthalimid)*, 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;
V. eine Mischung aus 3 Gew.-Teilen der Verbindung (N-[4-Chlor-5-(2-chlor-2-ethoxycarbonylethyl-1)-phenyl]-3,4,5,6-tetrahydrophthalimid)* und 97 Gew.-Teilen feinteiligem Kaolin. Dieses Stäubemittel enthält 3 Gew.-% Wirkstoff;
VI. eine stabile ölige Dispersion aus 20 Gew.-Teilen der Verbindung (N-[4-Chlor-3-(2-chlor-2-ethoxycarbonyl-ethenyl)-phenyl]-3,4,5,6-tetrahydrophthalisoimid)*, 2 Gew.-Teilen des Calciumsalzes der Dodecylbenzolsulfonsäure, 8 Gew.-Teilen Fettalkohol-polyglykolether, 2 Gew.-Teilen des Natriumsalzes eines Phenolsulfonsäure-harnstoffformaldehyd Kondensates und 68 Gew.-Teilen eines paraffinischen Mineralöls;

* Die Verbindung ist nicht erfindungsgemäß, steht jedoch beispielhaft für erfindungsgemäße Verbindungen
* Die Verbindung ist nicht erfindungsgemäß, steht jedoch beispielhaft für erfindungsgemäße Verbindungen
* Die Verbindung ist nicht erfindungsgemäß, steht jedoch beispielhaft für erfindungsgemäße Verbindungen

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

In Anbetracht der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rüben |
| Camellia sinensis | Teestrauch |
| Carthamus tinctorius | Saflor-Färberdistel |

| | |
|---|---|
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können aber auch nichtphytotoxische Öle und ölkonzentrate zugesetzt werden.

### Herstellungsbeispiele

### Beispiel 1

### N-[4-Chlor-5-(2-chlor-2-ethoxycarbonylethyl-1)-2-fluorphenyl]-3,4,5,6-tetrahydrophthalimid (Verbindung I.02)

Zu einer Suspension von 1,9 g tert.-Butylnitrit, 260 g Acrylsäureethylester, 1,9 g H₂O-freiem Kupfer(II)chlorid in 200 ml abs. Acetonitril tropfte man bei 0°C 3,5 g N-(5-Amino-4-chlor-2-fluorphenyl)-3,4,5,6-tetrahydrophthalimid in 5 ml abs. Acetonitril. Anschließend erwärmte man langsam auf Raumtemperatur und rührt bei dieser Temperatur 10 Std. nach. Nun gab man 40 ml verd. Salzsäure zu und extrahierte mit Methyl-tert.-butylether dreimal. Die vereinigten organischen Phasen wurden über Natriumsulfat getrocknet und eingeengt. Der Rückstand wurde an Kieselgel chromatographiert (Eluent: Petrolether:Diethylether = 2:1).
Ausbeute: 1,3 g (26 %); ¹H-NMR (d⁶-DMSO/TMS) : δ = 1,18 ppm (t, 3H); 1,74 (bs, 4H); 2,38 (bs,4H); 3,30 (dd, 1H); 3,47 (dd, 1H); 4,17 (q, 2H); 4,82 (dd, 1H); 7,51 (d, 1H); 7,84 (d, 1H).
* Die Verbindung ist nicht erfindungsgemäß, steht jedoch beispielhaft für erfindungsgemäße Verbindungen

### Beispiel 2

### (N-[3-(2-Brom-2-ethoxcarbonylethyl-1)-4-chlorphenyl]-3,4,5,6-tetrahydrophthalimid)*

Zu einer Suspension von 6,2 g tert.-Butylnitrit, 400 ml Acrylsäureethylester, 11,2 g wasserfreiem Kupfer(II)bromid in 200 ml abs. Acetonitril tropft man bei 0°C 11,6 g N-(3-Amino-4-chlorphenyl)-3,4,5,6-tetrahydrophthalimid in 20 ml abs. Acetonitril zu. Anschließend erwärmt man langsam auf Raumtemperatur und rührt bei dieser Temperatur 10 Std. nach. Nun gibt man 300 ml 20 %ige Salzsäure zu und extrahiert dreimal mit Diethylether. Die vereinigten org. Phasen werden über Na₂SO₄ getrocknet und eingeengt. Der Rückstand wird an Kieselgel chromatographiert (Eluent: Petrolether:Diethylether = 2:1). Ausbeute: 5,0 g (27 %); ¹H-NMR (d⁶-DMSO/TMS): δ = 1,17 (t, 3H); 1,75 (bs, 4); 2,35 (bs, 4H); 3,38 (dd, 1H); 3,55 (dd, 1H); 4,18 (q, 2H); 4,75 (t, 1H); 7,30 (dd, 1H); 7,38 (d, 1H); 7,60 (d, 1H).
* Die Verbindung ist nicht erfindungsgemäß, steht jedoch beispielhaft für erfindungsgemäße Verbindungen

### Beispiel 3

### (N-[4-Chlor-3-(2-chlor-2-ethoxycarbonylethyl-1-)-phenyl]-4-methyl- 3,4,5,6-tetrahydrophthalimid)*

### 1. Stufe (2-Chlor-2-ethoxycarbonylethyl-1-)-2-chlor-5-nitrobenzol):

Zu einer Suspension aus 6,5 g tert.-Butylnitrit, 6,0 g Acrylsäureethylester, 6,7 g wasserfreiem Kupfer-II-chlorid in 150 ml abs. Acetonitril wurden bei 20-25°C portionsweise 7,2 g 2-Chlor-5-nitroanilin gegeben. Nach 10 Std. Rühren bei 20-25°C tropfte man 100 ml 10 gew.-%ige Salzsäure zu. Die erhaltene Reaktionsmischung wurde schließlich dreimal mit Methyl-tert.-butylether extrahiert, über Natriumsulfat getrocknet und dann eingeengt. Der Rückstand wurde chromatographisch (an Kieselgel) gereinigt. Ausbeute: 3,8 g (31 %) ; ¹H-NMR (in d⁶-DMSO/TMS) : δ [ppm] = 1,28 (t, 3H), 3,36 (dd, 1H), 3,58 (dd; 1H), 4,24 (q, 2H), 4,56 (dd; 1H), 7,54 (d; 1H), 8,10 (dd; 1H), 8,18 (dd; 1H).

### 2. Stufe (3-(2-Chlor-2-ethoxycarbonyl-ethyl-1-)-4-chloranilin):

Zu einer Suspension aus 1,8 g Eisenpulver in 17,5 ml Ethanol und 9 ml Eisessig wurden bei 65°C portionsweise 2,92 g (2-Chlor-2-ethoxycarbonyl-ethyl-1)-2-chlor-5-nitrobenzol gegeben. Nach 3 Std. erhitzen auf Rückflußtemperatur kühlte man auf 20-25°C ab und versetzt die Reaktionsmischung mit Essigsäureethylester. Danach wurde der Feststoffanteil abgetrennt. Der Niederschlag wurde abfiltriert und das Filtrat eingeengt. Man nahm nochmals in Essigsäureethylester auf und wusch mit Wasser, trocknete über Natriumsulfat und engte ein. Ausbeute: 2,0 g (76 %); ¹H-NMR (in d⁶-DMSO/TMS): δ [ppm] = 1,12 (t,3H), 3,05 (dd, 1H), 3,22 (dd, 1H), 4,12 (dd,1H), 4,62 (t,2H), 5,25 (s, 2H), 6,48 (m, 2H), 7,01 (d, 1H).

### 3. Stufe (N-(4-Chlor-3-(2-chlor-2-ethoxycarbonylethyl-1-)-phenyl]-4-methyl-3,4,5,6-tetrahydrophthalimid):

2,60 g 3-(2-Chlor-2-ethoxycarbonylethyl-1-)-4-chloranilin und 1,65 g 4-Methyl-3,4,5,6-tetrahydrophthalsäureanhydrid wurden 6 Std. unter Rückfluß erhitzt. Nach Abkühlen und Einengen wurde der Rückstand in Essigsäureethylester aufgenommen und dreimal mit Wasser gewaschen. Nach Trocknen der org. Phase über Na₂SO₄ engte man ein. Ausbeute 3,0 g (75 %); ¹H-NMR (in d⁶-DMSO/TMS): δ [ppm] = 0,96 (d; 3H), 1,05 (t; 3H), 1,64-2,08 (m; 3H), 2,15-2,70 (m; 4H), 3,20-3,55 (m; 2H), 4,14 (q; 2H), 4,78 (dd; 1H), 7,26 (d; 1H), 7,34 (s; 1H), 7,55 (d; 1H).

In Tabelle I sind besonders bevorzugte substituierte Tetrahydrophthalimide I aufgeführt, die entsprechend den Beispielen 1 bis 3 hergestellt wurden.

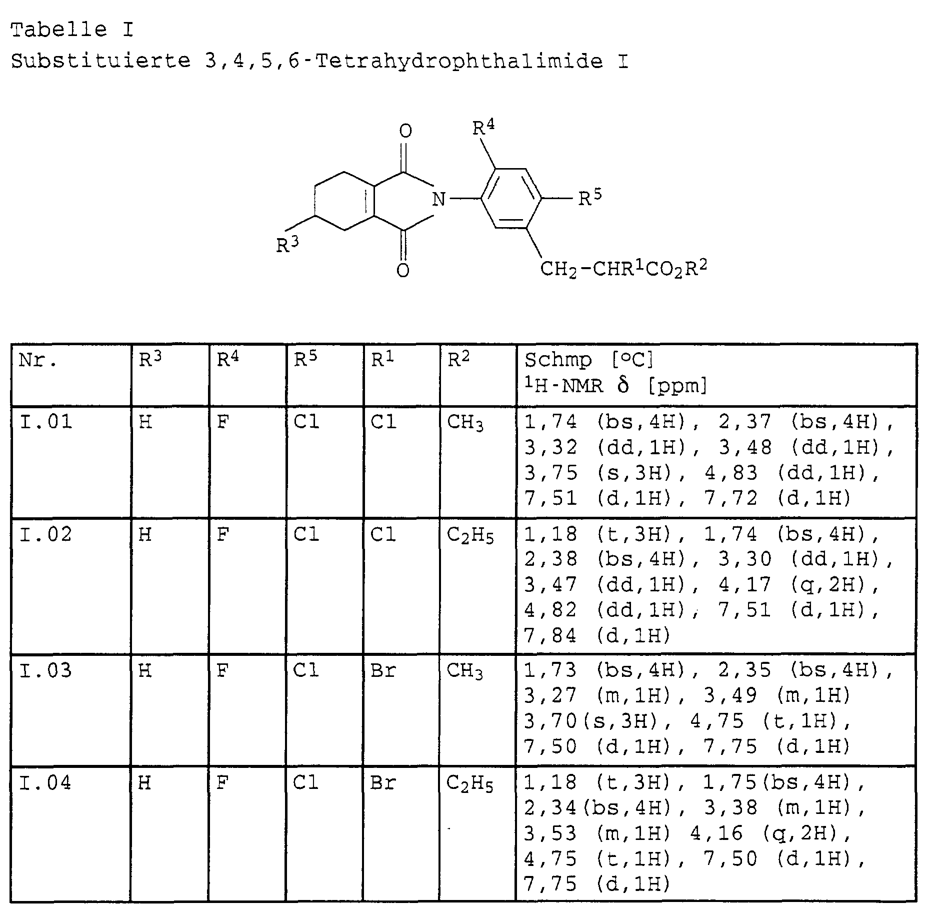

### Anwendungsbeispiele (herbizide Wirksamkeit)

Die herbizide Wirkung der substituierten Tetrahydrophthalimide I ließ sich durch Gewächshausversuche zeigen:

Als Kulturgefäße dienten Plastikblumentöpfe mit lehmigem Sand mit etwa 3,0 Gew.-% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkte ein gleichmäßigeres Keimen der Testpflanzen, sofern dies nicht durch die Wirkstoffe beeinträchtigt wurde.

Zum Zwecke der Nachauflaufbehandlung wurden die Testpflanzen je nach Wuchsform bis zu einer Wuchshöhe von 3 bis 15 cm angezogen und erst dann mit den in Wasser suspendierten oder emulgierten Wirkstoffen behandelt. Die Testpflanzen wurden entweder bereits in den Versuchsgefäßen gesät und aufgezogen, in denen sie behandelt wurden, oder sie wurden als Keimpflanzen getrennt angezogen und einige Tage vor der Behandlung mit den Wirkstoffaufbereitungen in die Versuchsgefäße verpflanzt.

Die Aufwandmenge für die Nachauflaufbehandlung betrug 0,5 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Amaranthus retroflexus | Zurückgekrümmter Fuchsschwanz | redroot pigweed |
| Centaurea cyanus | Kornblume | cornflower |
| Galium aparine | Klettenlabkraut | catchweed bedstraw |
| Ipomoea subspecies | Prunkwindearten | morningglory |

Das Ergebnis zeigte, daß mit der Verbindung Nr. I.02 unerwünschte Unkräuter sehr gut bekämpft werden können.

## Patentansprüche

1. Substituierte 3,4,5,6-Tetrahydrophthalimide der allgemeinen Formel I 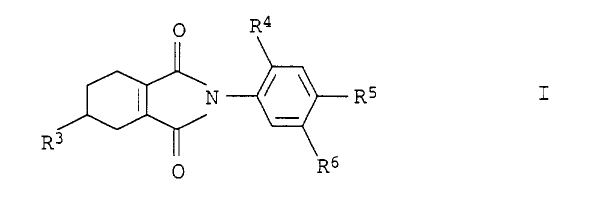 in der die Variablen folgende Bedeutung haben:
R³ Wasserstoff oder eine Methylgruppe;
R⁴ Fluor;
R⁵ Chlor;
R⁶ -CH₂-CHR¹-CO₂R²;
R¹ Chlor, Brom;
R² Methyl, Ethyl;
sowie deren umweltverträgliche Salze.

2. Verfahren zur Herstellung von substituierten 3,4,5,6-Tetrahydrophthalimiden der Formel I gemäß Anspruch 1, dadurch gekennzeichnet, daß man eine Verbindung der Formel III 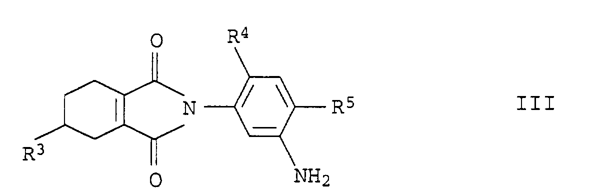 und eine Verbindung der Formel IV 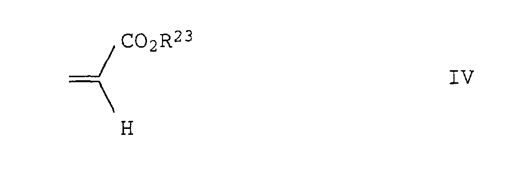 einer Meerwein'schen Arylierungsreaktion unterwirft.

3. Verfahren zur Herstellung von substituierten 3,4,5,6-Tetrahydrophthalimiden der Formel I gemäß Anspruch 1, dadurch gekennzeichnet, daß man eine Verbindung der Formel V hydriert

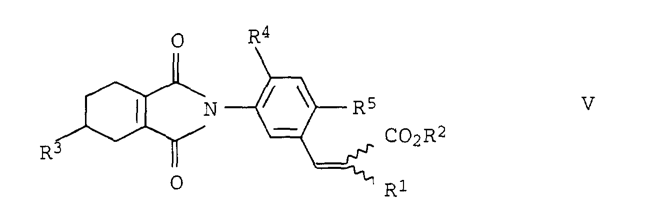

4. Verfahren zur Herstellung von substituierten 3,4,5,6-Tetrahydrophthalimiden der Formel I gemäß Anspruch 1, dadurch gekennzeichnet, daß R¹ durch ein anderes Halogen ersetzt wird.

5. Verfahren zur Herstellung von substituierten 3,4,5,6-Tetrahydrophthalimiden der Formel I gemäß Anspruch 1, dadurch gekennzeichnet, daß man ein Anilin der Formel VI 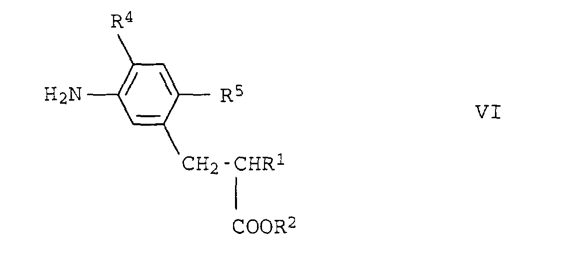 mit einem Tetrahydrophthalsäureanhydrid der Formel II 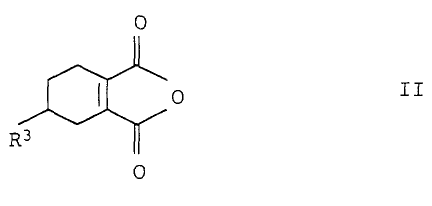 umsetzt.

6. Verfahren zur Herstellung von substituierten Anilinen der Formel VI gemäß Anspruch 5, dadurch gekennzeichnet, daß man eine Nitroverbindung der Formel VII 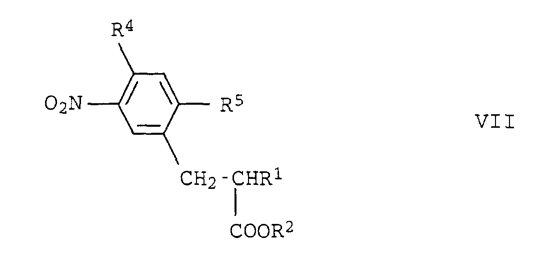 reduziert.

7. Verfahren zur Herstellung von substituierten Nitroverbindungen der Formel VII gemäß Anspruch 6, dadurch gekennzeichnet, daß man eine Verbindung der Formel VIII 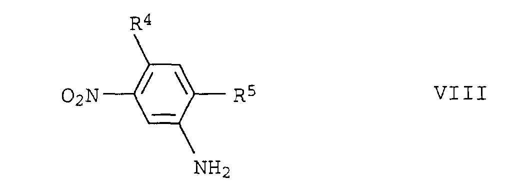 und eine Verbindung der Formel IV 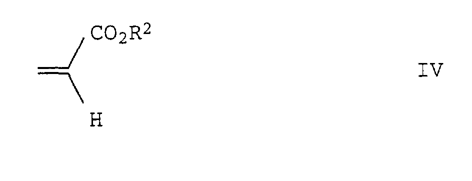 einer Meerwein'schen Arylierungsreaktion unterwirft.

8. Herbizides Mittel, enthaltend ein 3,4,5,6-Tetrahydrophthalimid der Formel I oder dessen umweltverträgliche Salze gemäß Anspruch 1, sowie übliche inerte Zusatzstoffe.

9. Mittel zur Desikkation und/oder Defoliation von Pflanzen enthaltend ein 3,4,5,6-Tetrahydrophthalimid der Formel I oder dessen umweltverträgliche Salze gemäß Anspruch 1, sowie übliche inerte Zusatzstoffe.

10. Verfahren zur Bekämpfung unerwünschten Pflanzenwuchses und zur Desikkation und/oder Defoliation von Pflanzen, dadurch gekennzeichnet, daß man ein 3,4,5,6-Tetrahydrophthalimid der Formel I oder dessen umweltverträgliches Salz, gemäß Anspruch 1, sowie übliche inerte Zusatzstoffe auf die Pflanzen und/oder deren Lebensraum einwirken läßt.

## Claims

1. A substituted 3,4,5,6-tetrahydrophthalimide of the general formula I 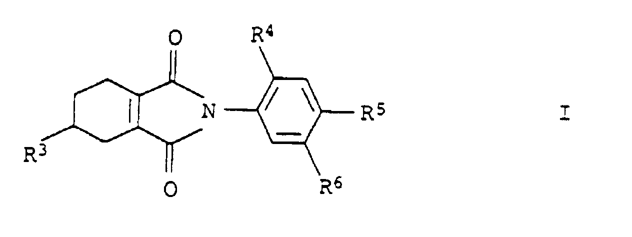 where the variables have the following meanings:
R³ is hydrogen or a methyl group;
R₄ is fluorine;
R₅ is chlorine;
R⁶ is -CH₂-CHR¹-CO₂R²;
R¹ is chlorine, bromine;
R² is methyl, ethyl;
and their environmentally tolerable salts.

2. A process for preparing substituted 3,4,5,6-tetrahydrophthalimides of the formula I as claimed in claim 1, which comprises subjecting a compound of the formula III 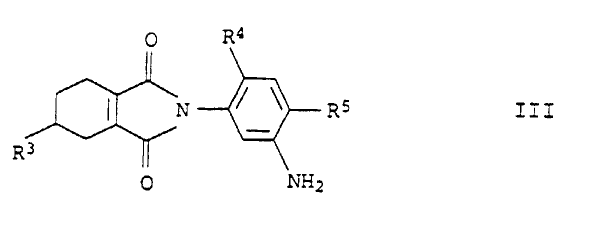 and a compound of the formula IV 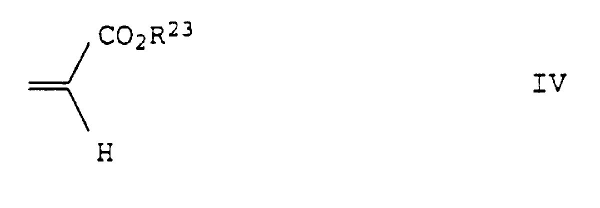 to a Meerwein arylation reaction.

3. A process for preparing substituted 3,4,5,6-tetrahydrophthalimides of the formula I as claimed in claim 1, which comprises hydrogenating a compound of the formula V

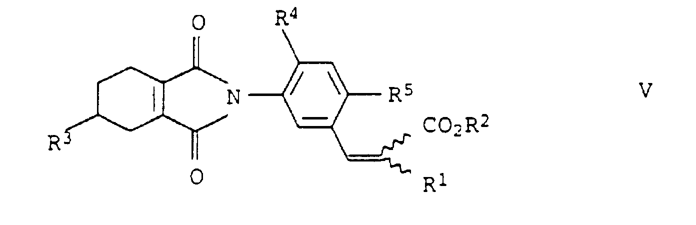

4. A process for preparing substituted 3,4,5,6-tetrahydrophthalimides of the formula I as claimed in claim 1, which comprises replacing R¹ by a different halogen.

5. A process for preparing substituted 3,4,5,6-tetrahydrophthalimides of the formula I as claimed in claim 1, which comprises reacting an aniline of the formula VI 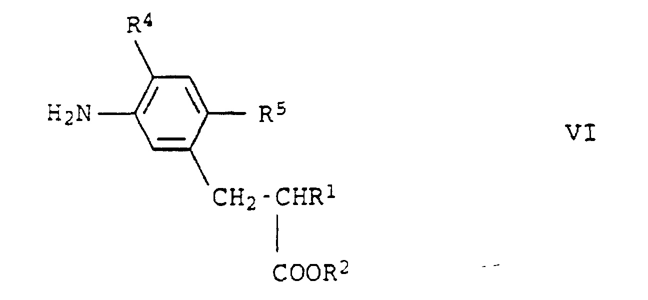 with a tetrahydrophthalic anhydride of the formula II

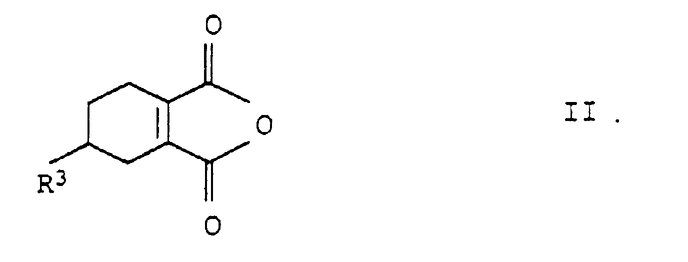

6. A process for preparing substituted anilines of the formula VI as claimed in claim 5, which comprises reducing a nitro compound of the formula VII

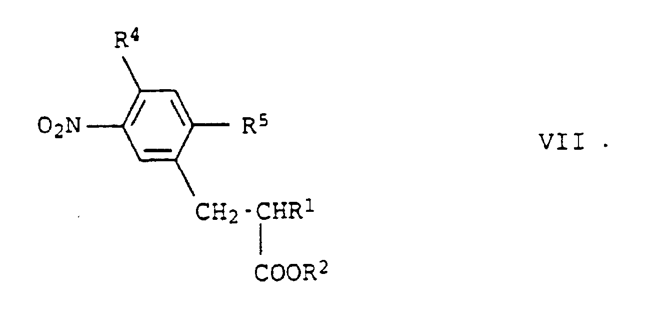

7. A process for preparing substituted nitro compounds of the formula VII as claimed in claim 6, which comprises subjecting a compound of the formula VIII 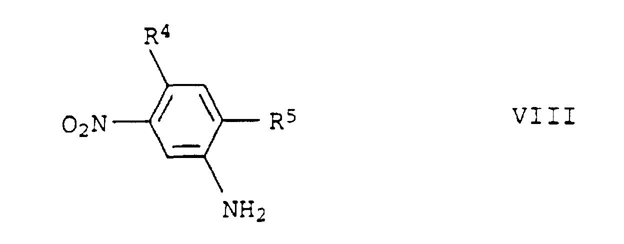 and a compound of the formula IV 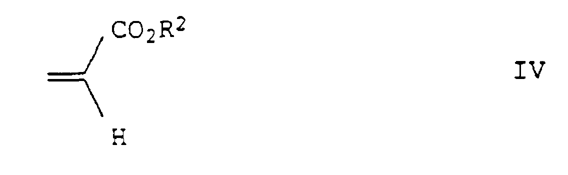 to a Meewein arylation reaction.

8. A herbicidal composition containing a 3,4,5,6-tetrahydrophthalimide of the formula I or its environmentally tolerable salts, as claimed in claim 1, and customary inert additives.

9. A composition for the desiccation and/or defoliation of plants, containing a 3,4,5,6-tetrahydrophthalimide of the formula I or its environmentally tolerable salt, as claimed in claim 1, and customary inert additives.

10. A process for controlling undesired plant growth and for the desiccation and/or defoliation of plants, which comprises allowing a 3,4,5,6-tetrahydrophthalimide of the formula I or its environmentally tolerable salt, as claimed in claim 1, and customary inert additives to act on the plants and/or their habitat.

## Revendications

1. 3,4,5,6-tétrahydrophtalimides substitués de formule générale I 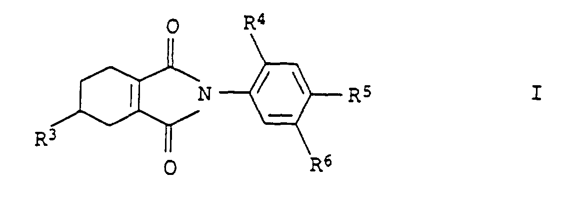 dans laquelle les symboles ont les significations suivantes :
R³ : l'hydrogène ou un groupe méthyle ;
R⁴ : le fluor ;
R⁵ : le chlore ;
R⁶ : un groupe -CH₂-CHR¹-CO₂R²;
R¹ : le chlore, le brome ;
R² : un groupe méthyle, éthyle ;
et leurs sels non polluants.

2. Procédé de préparation des 3,4,5,6-tétrahydrophtalimides substitués de formule I de la revendication 1, caractérisé par le fait que l'on soumet un composé de formule III 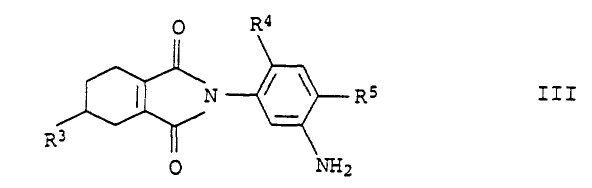 et un composé de formule IV 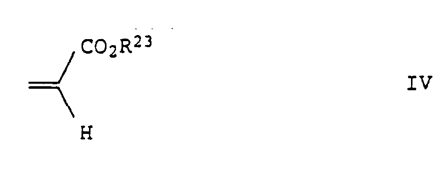 à une réaction d'arylation de Meerwein.

3. Procédé de préparation des 3,4,5,6-tétrahydrophtalimides substitués de formule I de la revendication 1, caractérisé par le fait que l'on hydrogène un composé de formule V

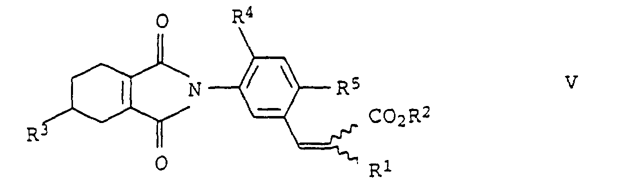

4. Procédé de préparation des 3,4,5,6-tétrahydrophtalimides substitués de formule I de la revendication 1, caractérisé par le fait que l'on remplace R1 par un autre halogène.

5. Procédé de préparation des 3,4,5,6-tétrahydrophtalimides substitués de formule I selon la revendication 1, caractérisé par le fait que l'on fait réagir une aniline de formule VI 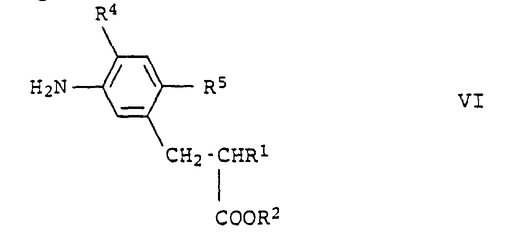 Avec un anhydride tétrahydrophtalique de formule II

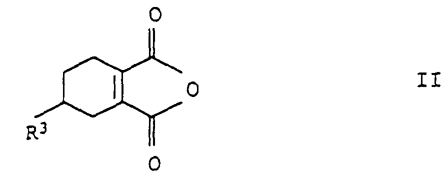

6. Procédé de préparation des anilines substituées de formule VI de la revendication 5, caractérisé par le fait que l'on réduit un dérivé nitré de formule VII

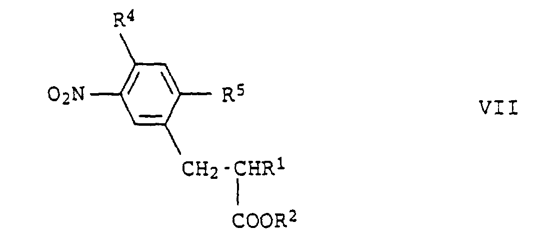

7. Procédé de préparation des dérivés nitrés substitués de formule VII de la revendication 6, caractérisé par le fait que l'on soumet un composé de formule VIII 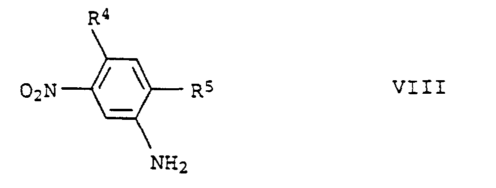 et un composé de formule IV 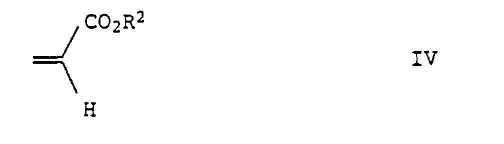 à une réaction d'arylation de Meerwein.

8. Produit herbicide contenant un 3,4,5,6-tétrahydrophtalimide de formule I ou ses sels non polluants selon la revendication 1, avec des additifs inertes usuels.

9. Produit pour la dessiccation et/ou la défoliation de végétaux contenant un 3,4,5,6-tétrahydrophtalimide de formule I ou ses sels non polluants selon la revendication 1, et des additifs inertes usuels.

10. Procédé pour combattre les croissance de végétaux indésirables ainsi que pour la dessiccation et/ou la défoliation des végétaux, caractérisé par le fait que l'on fait agir un 3,4,5,6-tétrahydrophtalimide de formule I ou l'un de ses sels non polluants selon la revendication 1, et des additifs inertes usuels, sur les végétaux et/ou leur habitat.
